# EUROPEAN PATENT SPECIFICATION

(11) **EP 1 727 508 B1**
(45) Date of publication and mention of the grant of the patent: **02.11.2011**
(21) Application number: 05722066.7
(22) Date of filing: 21.03.2005
(51) Int. Cl.: A61K 6/083, A61K 6/093, A61K 8/89, A61Q 11/00, C09D 183/04, C08L 83/04

(54) **POLY(DIALKYLSILOXANE) FOR IMPROVING SURFACE OF DENTAL FILLINGS**
POLY(DIALKYLSILOXAN) ZUR VERBESSERUNG DER OBERFLÄCHEN VON ZAHNFÜLLUNGEN
POLY(DIALKYLSILOXANE) POUR AMELIORER LA SURFACE DE MATIERES D'OBTURATION DENTAIRE

(30) Priority: 19.03.2004 NL 4075907
(43) Date of publication of application: 06.12.2006
(73) Proprietor: Stichting Glass for Health, 2343 JX Oegstgeest (NL)
(72) Inventor: VAN DEN BOSCH, Willem, Frederik, NL-2343 JX Oegstgeest (NL); VAN DUINEN, Raimond, Nicolaas, Bruno, NL-2552 XV Den Haag (NL)
(74) Representative: van Westenbrugge, Andries
(86) International application number: PCT/NL2005/000212
(87) International publication number: WO 2005/089700

(56) References cited:
- EP-A- 1 101 484
- WO-A-93/00878
- WO-A-2004/108095
- US-A- 3 574 943
- US-A- 5 453 456
- US-A1- 2003 211 051

## Description

### FIELD OF THE INVENTION

The present invention relates to dental fillings having improved properties, said dental fillings being formed by filling dental cavities with a glass ionomer cement composition In particular, the invention relates to a particular poly(dialkylsiloxane) for use in improving the strength and the surface of such dental fillings. In addition, the invention relates to use in a particular poly(dislkylsiloxane) for other medical and non-medical applications, in particular as an additive for bone cement compositions.

### BACKGROUND OF THE INVENTION

Glass ionomer cements ("GIC"), also known in the art as fluoroaluminosilicate glass cements ("FAS"), are widely used for already a considerable period of time in clinical and dental applications, e.g. as a permanent filling material. Reference is made to US 4.376.835, US 5.063.257, US 5.453.456, US 5.552.485 and US 5,670,258.

WO 2004/108095 (priority 5 June 2003; published 16 December 2004) discloses an improved glass ionomer cement composition (also indicated as glass carbomer cement composition or "GCC" that can be obtained by treating a fluorosilicate glass powder with (a) a poly(dialkylsiloxane) having terminal hydroxyl groups, wherein the alkyl groups contain 1 to 4 carbon atoms, (b) an aqueous acid solution, and (c) separating the treated fluorosilicate glass powder from the aqueous acid solution.

US 2003/211051 discloses compositions and methods for treating surfaces of teeth with dicarboxy polysiloxanes.

US 3.574.943 discloses a polysiloxane, a pressure sensitive, adhesive polymer for use as a cavity liner for drilled-out carious cavities.

EP 1.101.484 A discloses dental fillings comprising fine particles coated with a polysiloxane.

WO 93/00878 discloses a sol gel of a tetraethoxysilicone for coating teeth.

In this patent application the term GIC is used and it is also to be understood to encompass GCC's.

Commercially available GIC's are for example KetacMolar® from 3M ESPE, Fuji IX® from GC Corp. and Ionofil Molar AC® of Voco GmbH.

Commercially, GIC's are supplied by the manufacturer in capsules together with a leaflet containing instructions how the GIC's are activated. Obviously, activation can also be established by providing the ingredients separately, adding them together and mixing them manually. The usual method that is employed by a dentist is filling a dental cavity with the activated GIC which is then cured, preferably by using ultrasound and/or heat.

However, dental fillings made of prior art GIC's still suffer from several disadvantages. For example, the strength (that is, flexural strength and compression strength), stiffness, hardness and solubility properties of the dental fillings that can be obtained by using the GIC's according to the prior art are often insufficient The surface of these fillings are also not very smooth with the result that they are difficult to polish. Another disadvantage of these fillings is their relatively low wear resistance due to the rather high water solubility of GIC's (conventional GIC's have relatively a too high solubility in aqueous media, in particular acidic aqueous media as can prevail in the mouth so that the abrasive resistance of the cured GIC is unsatisfactory). In addition, it appears that the known GIC's require a considerable curing time for acquiring an acceptable strength. Furthermore, the handleability of GIC's is insufficient. These materials are highly viscous liquids and are difficult to apply and to finish to a smooth filling. Consequently, there is still a need for dental fillings having an improved strength and surface.

It is therefore an object of the present invention to provide the skilled practitioner with a method which enables him or her to form dental fillings having an improved strength and surface.

### SUMMARY OF THE INVENTION

The inventors have surprisingly found that when a dental cavity is filled with a conventional GIC to form a dental filling followed by aftertreating the surface of the incompletely cured dental filling with a poly(dialkyl)siloxane results in a dental filling having *inter alia* an improved strength surface. The invention therefore relates to a poly(dialkylsiloxane) having terminal hydroxyl groups, wherein the alkyl groups contain 1 to 4 carbon atoms, for use in improving the strength and the surface of dental fillings that are based on glass ionomer cement compositions.

Alternatively, the invention relates to the use of a poly(dialkylsiloxane) having terminal hydroxyl groups, wherein the alkyl groups contain 1 to 4 carbon atoms, as active ingredient for the preparation of a filling composition for improving the strength and the surface of dental fillings that are based on glass ionomer cement compositions.

The invention further relates to a dental filling composition comprising a poly(dialkylsiloxane) having terminal hydroxyl groups, wherein the alkyl groups contain 1 to 4 carbon atoms and said dental filling composition for use in improving the strength and the surface of dental fillings that are based on glass ionomer cement compositions.

### DETAILED DESCRIPTION OF THE INVENTION

Dental fillings obtained according to the method of the invention appear to have an improved strength and a lower solubility in comparison to dental fillings that are made according to the prior art methods and are therefore less sensitive to abrasion and wear. In addition, the surface of cured dental fillings obtained according to the method of the invention appear to be very smooth which makes finishing off of the dental filling, e.g. by polishing much easier. Another important advantage of the invention is that the handleability of the GIC is improved: the skilled practitioner first applies the GIC to the cavity and then finishes the filling with the poly(dialkylsiloxane). The inventors assume that the poly(dialkylsiloxane) mixes with the top layer of the GIC and said mixed top layer appears to cure faster than the GIC alone.

After the poly(dialkylsiloxane) has been applied, the dental filling is cured, preferably by using ultrasound and/or heat. As is well known in the art, ultrasound apparatus used in dentistry also provide heat. Various heat sources can obviously be used, e.g. IR sources including heating lamps, a heated needle etc. However, it also within the scope of the present invention that the poly(dialkylsiloxane) is applied after the curing step or that the poly(dialkylsiloxane) is applied prior to as well after the curing step.

Preferably, the poly(dialkylsiloxane) is applied within a period of less than 5 minutes after that the cavity is filled with the GIC since curing of the GIC already occurs under ambient conditions. More preferably, this period is less than 3 minutes and in particular less than 1 minute.

According to the invention, the poly(dialkylsiloxane) may be linear or cyclic. It may further be a blend of different poly(dialkylsiloxane)s, e.g. a blend of a poly(dimethylsioxane) of high kinematic viscosity and a poly(dimethylsiloxane) of low kinematic viscosity. It is further preferred that the alkyl groups of the poly(dialkylsiloxane) are methyl groups. The kinematic viscosity is preferably in the range of 1 cSt to 100.000 cSt at 25°C 1 to 100.000 mm²/s], preferably 100 cSt to 10.000 cSt at 25°C 100 to 10.000 mm²/s], even more preferably 500 cSt to 5000 cSt at 25°C 100 to 10.000 mm²/s]. The best results are obtained with a viscosity of about 1000 cSt at 25°C [about 1000 mm²/s]. Obviously, the poly(dialkylsiloxane) according to the invention may constitute a plurality or a blend of poly(dialkylsiloxanes) having different viscosities, wherein the plurality or blend of the poly(dilalkylsiloxanes) are formulated in such a way that the viscosity of the plurality or blend of the poly(dilalkylsnloxanes) is about 1000 cSt at 25°C [about 1000 mm²/s].

The GIC's that can be used in this invention are for example those that are disclosed in US 4.376.835, US 5.063.257, US 5.453.456, US 5.552.485 and US 5.670.258, WO 2004/108095.

However, according to the present invention, it is preferred that the GIC is a GCC (as disclosed in WO 2004/108095) which is obtainable by treating a fluorosilicate, glass powder with: (a) a poly(dialkylsiloxane) having terminal hydroxyl groups, wherein the alkyl groups contain 1 to 4 carbon atoms, (b) an aqueous acid solution, and (c) separating the treated fluorosilicate glass powder from the aqueous acid solution.

The fluorosilicate glass powder particles are generally depleted of calcium at their surface such that the quotient of the atomic ratio Si/Ca at the surface of the powder particles and the atomic ratio Si/Ca in the core region is at least 2.0, preferably at least 3.0, and most preferably at least 4.0. The calcium content of the powder particles of the invention increases asymptotically from the surface to the core region.

The depth of the depletion zone depends on the conditions given in each individual case: However, the depletion zone preferably extends at least to a depth of 10 nm, more preferably to at least 20 nm, and most preferably to at least 100 nm. These ranges are particularly suited for use of the fluorosilicate glass powders in dentistry. For other purposes, e.g., for use in bone cements, the depletion zone may also be deeper and may be 200 to 300 nm, for example.

As is known in the art, the fluorosilicate glass powders are produced by surface treatment of glass powders having a composition corresponding to the core region of the powders. Upon surface treatment the number of silicon atoms per unit volume remains substantially constant. The actual change in the absolute number of atoms per unit volume of other types of atoms is therefore obtained by forming the quotient of the relative atom proportion with the percentage silicon proportion. The quotient of the atomic ratio Si/Ca at the surface and the atomic ratio Si/Ca in the core region therefore constitutes a useful value to characterize the fluorosilicate glass powders.

The surface measurement to determine Ca depletion of the glass powders is suitably carried out by photo electron spectroscopy for chemical analysis (ESCA). This method has been described by R. S. Swingle II and W. M. Riggs in Critical Reviews in Analytical Chemistry, Vol. 5, Issue 3, pages 267 to 321, 1975 and by K. Levsen in "Chemie in unserer Zeit", Vol. 40, pages 48 to 53, 1976. The measuring data underlying the description presented above are outlined in US 4.376.835.

The fluorosilicate glass powders have an average particle size (weight average) of at least 0.01, preferably at least 0.5 µm, more preferably at least 1.0 µm, and most preferably at least 3.0 µm. For dental purposes the average particle size (weight average) is 0.01 to 50.0 µm, preferably 1.0 to 20.0 µm, more preferably 3.0 to 15.0 µm, most preferably 3.0 to 10.0 µm. The particles have a maximum particle size of 200 µm, preferably 150 µm, more preferably 100 µm, especially 60 µm. For use as dental bonding cement or as additive for bone cement compositions, the maximum particle size is 25 µm, preferably 20 µm. In order to achieve good mechanical properties a not excessively narrow particle size distribution is favorable, as usual, which is achieved, for example, by conventional grinding and classifying of the coarse.

The fluorosilicate glass powders are prepared from glass powders having the average composition of the core region of the powders described herein. To this end the glass powders described, for example, in DE A 2.061.513 and in Table 1 are suitable. The glass powders employed as starting materials are obtained as usual by fusing the starting components together at temperatures above 950°C., quenching, and grinding. The starting components may be, for example, the compounds stated in DE A 2.061.513 in suitable quantitative ranges.

The thus obtained powders are then subjected to a surface treatment. The powders are obtainable, for example, by removal of Ca by suitable chemical agents.

For example, the starting glass powders are treated on the surface with acid, preferably at room temperature. To this end substances containing acidic groups are employed, preferably substances forming soluble calcium salts. Sparing water-solubility of the respective calcium salts may be compensated to a certain degree by a large amount of liquid per unit of powder. The reaction period varies between a few minutes and several days, depending on the strength and concentration of the acid employed.

Thus, for instance, for the preparation of the powders hydrochloric, sulfuric, nitric, acetic, propionic and perchloric acid may be used.

The acids are employed at a concentration of 0.005 to 50% by weight, preferably from 0.01 to 10% by weight, more preferably from 0.05 to 3% by weight.

After the respective reaction period the powders are separated from the solution and thoroughly washed to leave substantially no soluble calcium salts on the surface of the powder particles. Finally the powder is dried, preferably above 70°C, and screened to the desired particle size ranges.

The stronger the acid employed and the longer a given acid acts on the powder the longer will be the processing period after mixing with the mixing fluid.

The favorable surface character of the powders permits the use of an especially high powder/fluid ratio in the cement mix resulting in high strength values of the hardened material. The possibility of using a particularly reactive mixing fluid has the same effect. Furthermore, the processing period of a cement of the invention may be tailored to meet the user's requirements. The length of the processing period hardly influences the subsequent hardening period, so that also upon long processing periods rapid setting and early water insensitivity occurs.

The glass powders may be mixed, to form dental cements or bone cements, with the conventional aqueous polycarboxylic acid solutions as described, for example, in DE A 2.061.513, DE A 2.439.882 and DE A 2.101.889. Suitable polycarboxylic acids are polymaleic acid, polyacrylic acid and mixtures thereof, or copolymers, especially maleic acid/acrylic acid copolymers and/or acrylic acid/itaconic acid copolymers. It is self-evident that with the use of an extremely reactive glass powder a less reactive polycarboxylic acid will be employed in order to obtain a satisfactory hardening characteristic.

In order to accelerate and improve hardening of said glass ionomer cements chelating agents may be added during mixing, in a manner known from DE A 2.319.715. Instead of the customary use of the aqueous polycarboxylic acid solution as mixing fluid, the glass powder may also be pre-mixed in the corresponding ratio with the dry powdered polycarboxylic acid, as the solid substances do not undergo reaction with each other. In that case water is used as mixing fluid, preferably an aqueous solution of a chelating agent together with conventional additives such as bacteriostatic agents, if appropriate.

In order to avoid metering errors and to attain optimum mechanical properties, the powders may be used in pre-dosaged form. For example, the glass powder is metered out in plastic containers. Then the cement can either be mixed mechanically within said plastic capsules, or the container may be emptied and the mix prepared by hand. The aqueous polycarboxylic acid solution in such a case is metered, for example, with a dripping bottle or with a syringe. The use of the powder of the invention in so-called shaker capsules, e.g., corresponding to DE A 2.324.296, is suitable. A predetemined quantity of powder is held in readiness in a so-called main compartment, while the fluid is contained in a separate cushion beneath a lateral clip. By exerting pressure on said clip the fluid is sprayed through a bore into the main compartment and is then available for mechanical mixing. In both types of capsules the pure glass powder may be replaced by a mixture of glass powder and dry polycarboxylic acid in predetermined quantities. The fluid component is then water or an aqueous solution of a chelating agent.

The use of the mixture of glass powder and dry polycarboxylic acid is especially advantageous if said mixture is pelletised. To this end, the dry polycarboxylic acid is used in finely divided form after removal of coarse portions. After thorough blending of said polycarboxylic acid powder with the glass powder pellets may be made in a conventional pelletising machine. The compacting pressure must be selected such that after the addition of the mixing fluid (water or aqueous tartaric acid solution, for example), the pellets can still be readily worked into a cement while, on the other hand, they possess sufficient mechanical stability for transportation. Pellets made in this way permit especially simple mixing into a cement paste after brief dissolution, e.g., in the corresponding amount of tartaric acid solution. The mixing fluid may be added, for example, from a drip bottle or from a syringe.

The particles of the fluorosilicate glass powder have preferably an average size of 0.5 µm to 200 µm, more preferably 3 µm to 150 µm, even more preferably 3 µm to 100 µm and in particular 20 µm to 80 µm.

It is preferred that the aqueous acid solution comprises an inorganic acid or an organic acid. It is even more preferred that the aqueous acid solution comprises an organic acid, wherein the organic acid is preferably a polymer, e.g. a polyacrylic acid. The aqueous acid solution has preferably a pH in the range of 2 to 7.

The poly(dialkylsiloxane) according to the present invention has also been found to be a suitable and beneficial additive in bone cement compositions that are used to restore, strengthen, repair or replace bone structures within a mammal. Such bone structures are found in bone as well as in teeth. As is known in the art, bone damages can occur as a result of broken bone, surgically removed bone portions, destroyed bone, degraded bone, and brittle bone. The poly(dialkylsiloxane) can also be used as a coating material for bone implants or other formed objects to be implanted in bone structures.

## Claims

1. Poly(dialkylsiloxane) having terminal hydroxyl groups, wherein the alkyl groups contain 1 to 4 carbon atoms, for use in improving the strength and the surface of dental fillings that are based on glass ionomer cement compositions, said dental fillings already being formed by filling a dental cavity with a glass ionomer composition.

2. Poly(dialkylsiloxane) for use according to claim 1, wherein the poly(dialkylsiloxane) is linear or cyclic.

3. Poly(dialkylsiloxane) for use according to claim 1 or claim 2, wherein the alkyl groups of the poly(dialkylsiloxane) are methyl groups.

4. Poly(dialkylsiloxane) for use according to any one of claims 1 - 3, wherein the poly(dialkylsiloxane) has a kinematic viscosity in the range of 1 to 100.000 mm²/s [cSt] at 25°C.

5. Poly(dialkylsiloxane) for use according to any one of claims 1 - 4, wherein the dental fillings are already formed by filling a dental cavity with a glass ionomer composition, wherein said glass ionomer cement composition is obtainable by treating a fluorosilicate glass powder with:
(a) a poly(dialkylsiloxane) having terminal hydroxyl groups, wherein the alkyl groups contain 1 to 4 carbon atoms;
(b) an aqueous acid solution; and
(c) separation of the treated fluorosilicate glass powder from the aqueous acid solution.

6. Poly(dialkylsiloxane) for use according to claim 5, wherein the particles of the fluorosilicate glass powder have an average size of 0.01 to 200 µm.

7. Poly(dialkylsiloxane) for use according to claim 5 or claim 6, wherein the aqueous acid solution comprises an inorganic acid or an organic acid.

8. Poly(dialkylsiloxane) for use according to claim 7, wherein the organic acid is a polymer.

9. Poly(dialkylsiloxane) for use according to any one of claims 5 - 8, wherein the aqueous acid solution has a pH in the range of 2 to 7.

10. Use of a poly(dialkylsiloxane) having terminal hydroxyl groups, wherein the alkyl groups contain 1 to 4 carbon atoms, as active ingredient for the preparation of a filling composition for improving the strength and the surface of dental fillings that are based on glass ionomer cement compositions, said dental fillings already being forme by filling a dental cavity with a glass ionomer composition.

## Patentansprüche

1. Poly(dialkylsiloxan) mit endständigen Hydroxylgruppen, worin die Alkylgruppen 1 bis 4 Kohlenstoffatome enthalten, zur Verwendung der Verbesserung der Festigkeit und der Oberfläche von auf Glasionomerzementzusammensetzungen basierenden Zahnfüllungen, wobei diese Zahnfüllungen bereits durch Füllen eines Loches im Zahn mit einer Glasionomerzusammensetzung gebildet worden sind.

2. Poly(dialkylsiloxan) zur Verwendung nach Anspruch 1, worin das Poly-(dialkylsiloxan) linear oder cyclisch ist.

3. Poly(dialkylsiloxan) zur Verwendung nach Anspruch 1 oder Anspruch 2, worin die Alkylgruppen des Poly(dialkylsiloxans) Methylgruppen sind.

4. Poly(dialkylsiloxan) zur Verwendung nach einem der Ansprüche 1 bis 3, worin das Poly(dialkylsiloxan) eine kinematische Viskosität im Bereich von 1 bis 100.000 mm²/s [cSt] bei 25°C hat.

5. Poly(dialkylsiloxan) zur Verwendung nach einem der Ansprüche 1 bis 4, worin die Zahnfüllungen bereits durch Füllen eines Loches im Zahn mit einer Glasionomerzusammensetzung gebildet worden sind, worin die Glasionomerzementzusammensetzung durch Behandlung eines Fluorsilikatglaspulvers mit
(a) einem Poly(dialkylsiloxan) mit endständigen Hydroxylgruppen, worin die Alkylgruppen 1 bis 4 Kohlenstoffatome enthalten,
(b) einer wässrigen Säurelösung, und
(c) Trennung des behandelten Fluorsilikatglaspulvers von der wässrigen Säurelösung
erhaltbar ist.

6. Poly(dialkylsiloxan) zur Verwendung nach Anspruch 5, worin die Teilchen des Fluorsilikatglaspulvers eine mittlere Größe von 0,01 bis 200 µm haben.

7. Poly(dialkylsiloxan) zur Verwendung nach Anspruch 5 oder 6, worin die wässrige Säurelösung eine anorganische Säure oder eine organische Säure umfasst.

8. Poly(dialkylsiloxan) zur Verwendung nach Anspruch 7, worin die organische Säure ein Polymer ist.

9. Poly(dialkylsiloxan) zur Verwendung nach einem der Ansprüche 5 bis 8, worin die wässrige Säurelösung einen pH-Wert im Bereich von 2 bis 7 hat.

10. Verwendung eines Poly(dialkylsiloxans) mit endständigen Hydroxylgruppen, worin die Alkylgruppen 1 bis 4 Kohlenstoffatome enthalten, als aktiver Wirkstoff für die Herstellung einer Füllzusammensetzung zur Verbesserung der Festigkeit und der Oberfläche von auf Glasionomerzementzusammensetzungen basierenden Zahnfüllungen, wobei diese Zahnfüllungen bereits durch Füllen eines Loches im Zahn mit einer Glasionomerzusammensetzung gebildet worden sind.

## Revendications

1. Poly(dialkylsiloxane) comportant des groupes hydroxyle terminaux, dans lequel les groupes alkyle contiennent 1 à 4 atomes de carbone, destiné à une utilisation servant à améliorer la résistance et la surface des matières d'obturation dentaire à base de compositions de ciment au verre ionomère, lesdites matières d'obturation dentaire étant déjà formées par remplissage d'une cavité dentaire avec une composition de verre ionomère.

2. Poly(dialkylsiloxane) destiné à une utilisation selon la revendication 1, dans lequel le poly(dialkylsiloxane) est linéaire ou cyclique.

3. Poly(dialkylsiloxane) destiné à une utilisation selon la revendication 1 ou la revendication 2, dans lequel les groupes alkyle du poly(dialkylsiloxane) sont des groupes méthyle.

4. Poly(dialkylsiloxane) destiné à une utilisation selon l'une quelconque des revendications 1 à 3, dans lequel le poly(dialkylsiloxane) présente une viscosité cinématique dans la plage de 1 à 100 000 mm²/s [cSt] à 25 °C.

5. Poly(dialkylsiloxane) destiné à une utilisation selon l'une quelconque des revendications 1 à 4, dans lequel les matières d'obturation dentaire sont déjà formées par obturation d'une cavité dentaire avec une composition de verre ionomère, dans lequel ladite composition de ciment au verre ionomère peut être obtenue par traitement d'une poudre de verre de fluorosilicate avec :
(a) un poly(dialkylsiloxane) comportant des groupes hydroxyle terminaux, où les groupes alkyle contiennent 1 à 4 atomes de carbone ;
(b) une solution aqueuse acide ; et
(c) une séparation de la poudre de verre de fluorosilicate traitée de la solution aqueuse acide.

6. Poly(dialkylsiloxane) destiné à une utilisation selon la revendication 5, dans lequel les particules de la poudre de verre de fluorosilicate présentent une taille moyenne de 0,01 à 200 µm.

7. Poly(dialkylsiloxane) destiné à une utilisation selon la revendication 5 ou la revendication 6, dans lequel la solution aqueuse acide comprend un acide inorganique ou un acide organique.

8. Poly(dialkylsiloxane) destiné à une utilisation selon la revendication 7, dans lequel l'acide organique est un polymère.

9. Poly(dialkylsiloxane) destiné à une utilisation selon l'une quelconque des revendications 5 à 8, dans lequel la solution aqueuse acide a un pH dans la plage de 2 à 7.

10. Utilisation d'un poly(dialkylsiloxane) comportant des groupes hydroxyle terminaux, dans lequel les groupes alkyle contiennent 1 à 4 atomes de carbone, en tant qu'ingrédient actif pour la préparation d'une composition d'obturation servant à améliorer la résistance et la surface de matières d'obturation dentaire à base de compositions de ciment au verre ionomère, lesdites matières d'obturation dentaire étant déjà formées par remplissage d'une cavité dentaire avec une composition de verre ionomère.
